# EUROPÄISCHE PATENTSCHRIFT

(11) **EP 1 502 571 B1**
(45) Veröffentlichungstag und Bekanntmachung des Hinweises auf die Patenterteilung: **08.04.2009**
(21) Anmeldenummer: 04014668.0
(22) Anmeldetag: 23.06.2004
(51) Int. Cl.: A61K 6/083

(54) **Dentalmaterialien mit hoher Abrasionsfestigkeit**
Dental material with a high abrasion resistance
Matériaux déntaires avec une haute résistance à l'abrasion

(30) Priorität: 30.07.2003 DE 10335181
(43) Veröffentlichungstag der Anmeldung: 02.02.2005
(73) Patentinhaber: Heraeus Kulzer GmbH, 63450 Hanau (DE)
(72) Erfinder: Erdrich, Albert, Dr., 61231 Bad Nauheim (DE); Stange, Frank-Uwe, 88085 Langenargen (DE); Herrmann, Cornelia, 88085 Langenargen (DE); Renz, Karl-Heinz, 71131 Jettingen (DE); Savic, Novica, 63691 Ranstadt (DE)
(74) Vertreter: Kühn, Hans-Christian

(56) Entgegenhaltungen:
- EP-A- 0 142 784
- EP-A- 0 442 326
- EP-A- 0 836 845
- EP-A- 1 230 906
- DE-A- 19 617 876
- DE-A- 19 848 886

## Beschreibung

Die Erfindung betrifft künstliche Zähne, die oder deren Schneidebereiche oder deren Schmelzbereiche aus einem Material mit hoher Abrasionsfestigkeit bestehen.
Dentalmaterialien mit einer Reihe verschiedener Füllstoffarten sind bekannt.

Z. B. wird in US 6063830 (Shofu, Kyoto; **A**) die Herstellung und Zusammensetzung eines Zahnmaterials beschrieben. Dieses besteht aus einer Kombination von in Urethandimethacrylat feinverteilter silanisierter Kieselsäure (SiO₂) mit Meth-/Acrylat-Monomeren und PMMA-Copolymeren. Beansprucht wird eine Menge von 10-70 % SiO₂, auf das eingesetzte Urethandimethacrylat bezogen. Beschrieben wird dabei eine hohe Abrasionsfestigkeit bei gleichzeitig erhöhter Zähigkeit.

Aus EP0962215 A2 (GC Dental Corp.; B) ist ein anorganische Füllstoffe enthaltendes Zahnmaterial bekannt. Neben verschiedenen Monomeren und vernetzten und unvernetzten Polymeren enthalten ist ein anorganisch-organischer Füllerkomplex (1). Optional enthalten sein kann ein weiterer anorganischer Füller (2). Zur Herstellung des Füllerkomplexes (1) werden anorganische Füllstoffe in meth-/acrylatbasierenden Monomeren gelöst und polymerisiert. Anschließend wird das Polymer mittels Kugelmühlen gebrochen und als vermahlendes Pulver eingesetzt. Die Füller (2) können verschiedene im Dentalbereich bekannte Gläser sein, wobei auf das Problem der mangelnden Plaqueresistenz hingewiesen und Silanisierung als Abhilfe empfohlen wird.

DE2462271 A1 (Ivoclar, Schaan; **C**) betrifft ein Material zur Herstellung dentaler Formkörper, unter anderem auch künstlicher Zähne. Verwendet als Füllstoff werden Siliziumdioxid und Aluminiumoxid in Korngrößen von 5 -700 nm und einer Menge von 10-90 %. Optional können die Füllstoffe silanisiert sein.

US5548001 (**D1**) und US4389507 (**D2**) (Heraeus Kulzer, bzw. Bayer AG) befassen sich mit der Herstellung von Peripolymeren mit Anteilen an anorganischen Füllstoffen. **D1** weist vermehrt auf die Herstellung mit vernetzenden Monomeren hin, während **D2** generell die Herstellmöglichkeiten von anorganisch verstärkten Polymerperlen beschreibt. Anwendungsmöglichkeiten oder Anwendungsgebiete werden nicht genannt.

US 4,617,327(**D3**) betrifft Füllstoffe mit anorganischem Kern, einer Schicht Vinylsilan und einer zweiten Schicht Methacrylatpolymer und deren Verwendung zur Herstellung von Brücken, Zähnen oder Füllungen mit hoher mechanischer Festigkeit und hoher Widerstandsfähigkeit gegen Abrieb (Spalte 4. Z. 19,20). Z.B. werden die Füller zusammen mit BisGMA und TEGDMA sowie Pigmenten zu künstlichen Zähnen verarbeitet.

In EP0677286 B1 (Heraeus Kulzer; **E**) wird Zahnmaterial beschrieben, welches Bariumaluminiumsilikatglas und mikrofeines Siliciumdioxid als anorganische Füllstoffe enthält. Dabei wird die Mischung hergestellt durch Zugabe der silanisierten Füllstoffe zu einer Matrix aus verschiedenen Meth-/acrylaten.

Zusammenfassend ergeben sich folgende Gemeinsamkeiten aus dem Stand der Technik:
■ Als Matrix werden verschiedene Arten von Meth-/acrylatmonomeren sowie organische Füllstoffe eingesetzt, die auch teilvernetzt sein können.
■ In der Regel wird hochdisperser Füllstoff, speziell Siliziumdioxid in optional silanisierter Form (**A, C, E**) zugegeben.
■ Vermahlenes Dentalglas, vorteilhafterweise silanisiert **(B, E),** wird als Füllstoff verwendet.

Relevante Merkmale einzelner Dokumente sind:
■ Verwendung eines Splitterpolymers als Füllstoff, d.h. anorganischer Füllstoff wird in Monomer vermischt, polymerisiert und gemahlen (**B**)
■ Verwendung von Perlpolymeren mit anorganischem Kern und Silanschicht + Methacrylatschicht: (**D3**)

Die bekannten Zusammensetzungen des Stands der Technik weisen verschiedene Nachteile auf. Dazu zählen:
■ Die direkte Zugabe von hochdispersen Füllstoffen, die automatisch große benetzbare Oberflächen aufweisen, führt zu:
   - schlechtem Handlingverhalten im Verlauf der Herstellung durch starkes Andicken, damit verbunden kann weniger Gesamtfüllstoff eingebracht werden bzw. es entstehen Inhomogenitäten.
   - hoher Affinität zu Plaqueanlagerungen und damit Verfärbungen.
■ Methacrylatfunktionalisierte (silanisierte) Füllstoffe erhöhen bei direkter Zugabe zu Monomer durch ihren hohen Grad an Oberflächenfunktionalität die Sprödigkeit des Materials.
■ Splitterpolymere wirken sich formbedingt sehr negativ auf das Handlingsverhalten während der Herstellung aus. Ergänzend kann es speziell bei Verwendung vernetzter Monomere später zu Verbundproblemen mit der Kunststoffmatrix kommen. Ein weiteres Problem sind rauhere Oberflächen durch die Splitterform der Füllstoffe.
■ Gemahlenes Dentalglas verschlechtert die Oberflächengüte deutlich, so daß aufwendigere Poliermethoden anzuwenden sind. Auch steigt die Härte des Materials stark an, was im Hinblick auf die Belastungen des Prothesenlagers und die Widerstandsfähigkeit gegen mechanische Belastungen ungünstig ist.

Ziel der vorliegenden Erfindung ist somit diese Nachteile zumindest teilweise zu beseitigen und insbesondere folgende Verbesserungen zu erreichen:
■ Schlechte Handlingseigenschaften bei Verwendung anorganischer Komponenten, speziell hochfeiner Füllstoffe oder auch Splitterpolymere sind zu verbessern. Das gilt sowohl für die Lagereigenschaften (Separation/Entmischung durch Dichteunterschiede im Pulver) als auch für die Mischeigenschaften, d.h. bei der Verarbeitung zu einer Mischung.
■ Eine hohe Oberflächengüte ohne aufwendige Politur sollte angestrebt werden
■ Verbundprobleme an der Trennschicht zwischen den Hals-/Dentin-/Schmelzschichten des künstlichen Zahns sind zu mildern.
■ Sprödigkeit und hohe Härte sind zugunsten von zähelastischem Verhalten bei gleich bleibender Abrasionsfestigkeit abzusenken.

Die Aufgabe wird durch
künstliche Zähne gelöst, die oder deren Schneidebereiche oder deren Schmelzbereiche aus einem Material mit hoher Abrasionsfestigkeit bestehen, das die folgende Zusammensetzung aufweist:

| | | |
|---|---|---|
| (a) | Monofunktionelle Meth-/acrylate | 25 - 30 % |
| (b) | Vernetzendes Meth-/acrylat | 6 - 10 % |
| (c) | Splitterpolymer aus Komponente (b) und (e) | 12 -18 % |
| (d) | PMMA-Perlpolymere, teilweise vernetzt | 15 - 25 % |
| (e) | Pyrogene Kieselsäure, silanisiert | 1 - 5 % |
| (f) | Anorganisch verstärktes Perlpolymer | 20 - 30 % |
| (g) | Initiatorkomponenten | 0,1 - 1 % |
| (h) | Farbpigmente | 0,1 - 3 % |

In den durchgeführten Versuchen zeigte sich entgegen den Erwartungen, daß sich durch ein ausgewogenes Verhältnis der Komponenten in Gegenwart einer speziell ausgestalteten Polymer-Vorstufe erheblich bessere Produkteigenschaften des fertigen Zahnmaterials erzielen lassen. Bei dieser Vorstufe handelt es sich um ein methacrylatbasierendes Perlpolymer, in welchem anorganisches Dentalglas als Füllstoff einpolymerisiert ist.

Die Mischungsbestandteile werden sofern nötig im folgenden näher erläutert:
Zu (a), (b) - Als monofunktionelle oder vernetzende¹ (Meth)acrylate kommen in Frage:Monofunktionelle oder polyfunktionelle (Meth)acrylate, die alleine oder in Mischungen eingesetzt werden können. Als Beispiele für diese Verbindungen kommen Methylmethacrylat, Isobutylmethacrylat, Cyclohexylmethacrylat, Triethylenglycoldimethacrylat, Diethylenglycoldimethacrylat, Tetraethylenglycoldimethacrylat, Ethylenglycoldimethacrylat, Polyethylenglycoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat, ethoxyliertes Bisphenol-A-dimethacrylat, aber auch Bis-GMA (2,2-bis-4-(3-methacryloxy-2-hydroxypropyl)-phenylpropan) sowie die Reaktionsprodukte aus Isocyanaten, insbesondere Di- und/oder Triisocyanaten und OH-gruppenhaltigen Methacrylaten, und die entsprechenden Acrylate aller obigen Verbindungen in Frage. Beispiele für Reaktionsprodukte von Isocyanaten sind die Umsetzungsprodukte von 1 Mol Hexamethylendiisocyanat mit 2 Mol 2-Hydroxyethylmethacrylat, von 1 Mol (Tri(6-isocyanatohexyl)biuret mit 3 Mol Hydroxyethylmethacrylat und von 1 Mol Trimethylhexamethylendiisocyanat mit 2 Mol Hydroxyethylmethacrylat, die auch als Urethandimethacrylate bezeichnet werden. Geeignete Monomere sind jeweils die Monomere selbst, daraus hergestellte polymerisierbare Präpolymere sowie Mischungen von diesen.
Bevorzugte Vemetzermonomere sind z.B. 2,2-Bis-4-(3-methacryloxy-2-hydroxypropyl)-phenypropan) (Bis-GMA), d.h. das Umsetzungsprodukt von Glycidylmethacrylat und Bisphenol-A (OH-gruppenhaltig), und 7,7,9-Trimethyl-4,13-dioxo-3,14-dioxa-5,12-diazahexadecan-1,16-diyl-dimethacrylat (UDMA), d.h. das Urethandimethacrylat aus 2 Mol 2-Hydroxyethylmethacrylat (HEMA) und 1 Mol 2-2,4-Trimethylhexamethylendiisocyanat (urethangruppenhaltig). Darüber hinaus sind Umsetzungsprodukte von Glycidylmethacrylat mit anderen Bisphenolen, wie z.B. Bisphenol-B (2,2'-Bis-(4-hydroxyphenyl)-butan), Bisphenol-F (2,2'-Methylendiphenol) oder 4,4'-Dihydroxydiphenyl, sowie Umsetzungsprodukte von 2 Mol HEMA oder 2-Hydroxypropyl(meth)acrylat mit, insbesondere 1 Mol, bekannter Diisocyanate, wie z.B. Hexamethylendiisocyanat, m-Xylylendiisocyanat oder Toluylendiisocyanat, als Vemetzermonomere bevorzugt.
¹Vernetzende Meth-/acrylate sind naturgemäß Verbindungen mit 2 oder mehr Metahcrylatgruppen im Momomeren

Zu (f) - Das anorganisch verstärkte Perlpolymer, das als Füllstoff eingesetzt wird, kann eines der in den oben erwähnten **D1, D2** und **D3** beschriebenen sein. Bevorzugt hat es folgende Zusammensetzung:

| | |
|---|---|
| Methylmethacrylat | 50 - 85 % |
| Monofunktionelles Methacrylat | 1-10 % |
| Silanisiertes Glas | 10 - 40 % |

Als monofunktionelle Monomere sollten vorteilhafterweise solche verwendet werden, die flexibilisierende Eigenschaften haben. Dieses sind unter anderem Alkylmeth-/acrylate wie Ethyl-MA, Butyl-Ma, Ethyl-Hexyl-MA, Methylacrylat.

Als silanisiertes Glas geeignet sind alle im Dentalbereich verwendeten Gläser, wie z.B. Quarzglas oder Barium-Aluminiumsilikatglas. Die mittlere Teilchengrösse sollte < 4 µm betragen, da bei größeren Teilchen die Abrasionseigenschaften (Herausreißen) und die Oberflächengüte leiden. Zu kleine Füllstoffe unter 100 nm zeigen keine signifikant erhöhten Abrasionswerte im Vergleich zu bekannten Werkstoffen

Die Herstellung der Füllstoffe erfolgt durch eine herkömmliche Suspensionspolymerisation. Dabei wird das Monomer in einer wasserbasierenden Phase unter Zugabe von Seife vordispergiert. Der anorganische Füllstoff wird zugegeben und in den Monomertröpfchen integriert. Durch Erwärmen der Mischung wird die Reaktion ausgelöst und die Tröpfchen zu Perlpolymeren ausgehärtet.

Vorteile dieses Folistoffes sind:
■ Die Matrix um die Füllstoffe und die Verbindungsfläche zwischen Matrix und Füllstoff ist sehr gut polymerisert, damit sehr widerstandsfähig gegen mechanische Belastungen.
■ Die Zugabe des flexibilisierenden Monomers ergibt eine zäh-elastische Matrix.
■ Dadurch, dass die Matrix nicht vernetzt ist, kann im Rahmen eines Quellvorganges problemlos eine Einbindung in eine übergeordnete Kunststoffmatrix erfolgen.
■ Der Füllstoff weist wegen der optimierten Grenzflächeneigenschaften eine nur geringe Trübung auf
■ Die Einkapselung der Füllstoffe ergibt sehr glatte Oberflächenstrukturen
■ Durch die kugelförmige Struktur ist das Mischverhalten beim Anteigen mit Monomer angenehm und leicht ein homogener Teig erzielbar.

Zu (g) Als Initiatoren kommen die dem Fachmann bekannten, für thermische Polymerisation geeigneten Initiatoren in Betracht. Bei der Auswahl des Initiators wird im allgemeinen die Polymerisationsneigung des Monomers und die Polymerisationsbedinggungen berücksichtigt. Wenn etwa ein Methacrylat bei hoher Temperatur polymerisiert wird, setzt man z.B. ein organisches Peroxid wie Benzoylperoxid ("BPO"), Di-tert-butylperoxide oder Cumolhydroperoxid, oder eine Azoverbindung wie 2,2-min-Azobisisobutyronitril oder 1,1-min-Azo-bis(cyclohexane-1-carbonitril). Für die Polymerisation bei Raumtemperatur wählt man zweckmäßig Redox Initiatoren, wie z.B. Benzoylperoxid/Dimethylanilin-Cumolhydroperoxid/Thioharnstoff, Ascorbinsäure/Cu Salz and Organische Sulfinsäure (oder Salz davon)/Amine/Peroxide oder auch Tributylboran, z.B. Benzoylperoxid mit einen aromatischen tertiären Amin oder Tributylboran-Partialoxid (TBBO).

Zu (h) Als Pigmente kommen die im Dentalbereich üblichen, dem Fachmann bekannten, in Betracht.

### Beispiel

Als vorteilhaft erwiesen hat sich folgende beispielhafte Ausgestaltung eines erfindungsgemäßen Materials:

| | | |
|---|---|---|
| (a) | Monofunktionelle Meth-/acrylate | 26 - 29,8 % |
| (b) | Vernetzendes Meth-/acrylat | 8 % |
| (c) | Splitterpolymer aus Komponente (b) und (e) | 15 % |
| (d) | PMMA-Perlpolymere, teilweise vernetzt | 20 % |
| (e) | Pyrogene Kieselsäure, silanisiert | 2 % |
| (f) | Anorganisch verstärktes Peripolymer | 25 % |
| (g) | Initiatorkomponenten | 0,1 - 1 % |
| (h) | Farbpigmente | 0,1 - 3 % |

### Herstellungsverfahren

Normale PMMA-basierende Materialien werden aus einer eingefärbten Pulverkomponente und einer den Initiator enthaltenden Flüssigkeitskomponente gemischt. Von großer Bedeutung ist dabei ein gutes Mischverhalten, was die Entstehung von Inhomogenitäten und Porositäten vermeidet. Anschließend kann der Teig unter Kühlung bis zur Polymerisation durch Wärme zwischengelagert werden.
Bei Verwendung der oben beschriebenen Zusammensetzung sind kaum Änderungen des bekannten Verfahrens notwendig. Durch die homogene Mischbarkeit und nicht so extremen Dichteunterschiede zwischen PMMA-Perlen und anorganisch gefüllten Perlen ist eine herkömmliche Einfärbung und Lagerung der Pulverkomponente möglich.
Lediglich optional eingesetzte pyrogene Kieselsäure ist separat in Monomer zu dispergieren und im Rahmen des Mischprozesses zuzugeben.

### Erläuterungen zu den künsflichen Zähnen

Künstliche Zähne werden zur Erzielung einer hochwertigen Ästhetik mindestens 2-schichtig, in der Regel jedoch 3-schichtig hergestellt (Dentin/Hals/Schneide bzw. Schmelz). Den höchsten mechanischen Belastungen ausgesetzt ist generell die äußere Schneide- bzw. Schmelzschicht. Insofern ist bei dieser Schicht eine hohe Abrasionsbeständigkeit die wichtigste Anforderung. Innerhalb der Schmelzschicht ist somit der bevorzugte Einsatzbereich des erfindungsgemäßen Materials
Die darunter liegenden Schichten Dentinschicht und Halsschicht bestehen in der Regel aus rein PMMA-basierendem Material, da hier eher eine möglichst einfache Anbindbarkeit an die aus PMMA bestehende Prothesenbasis von Wichtigkeit ist.

### Meßtechnische Ergebnisse

### Härtemessungen

Die Härte wird nach einer modifizierten Vickers-Härtemessung bestimmt. Im Gegensatz zur Messung der Eindruckdimensionen nach der Belastung wird bei der Zwick-Härtemessung direkt während des Belastens gemessen. Dieses eliminiert die Probleme der korrekten Messbarkeit von Proben mit elastischen Anteilen wie etwa Kunststoffen.

| **Material** | **Ergebnis Härtemessung (Zwickhärte HZ1) [N/mm²]** |
|---|---|
| PMMA-Zahnmaterial | 150 |
| PMMA-Zahnmaterial vernetzt mit ca. 8 - 15 % Vernetzer | 160 |
| PMMA-Zahnmaterial vernetzt, mit Splitterpolymer | 170 |
| PMMA-Zahnmaterial vernetzt, mit hochdispersern Füllstoff <100nm | 200 |
| PMMA-Zahnmaterial vernetzt, mit Dentalglas 0,5 - 3 µm | 300 - 400 abhängig vom Typ |
| Mitbewerber Hard-Resin-Tooth Endura (SHOFU) | 170 - 180 |
| Vergleichscomposit Dentacolor Sirius (HERAEUS KULZER) | 220 |
| Gemäß Erfindung | 140 |

### Biegefestigkeit, E-Modul, Schlagzähigkeit

Die Biegefestigkeit und das E-Modul wurden anhand der EN ISO 1567 - Prothesenkunststoffe bestimmt, die Schlagzähigkeit nach DIN 53435.

| | **PMMA** | **Vergleichskomposit** | **Gemäß Erfindung** |
|---|---|---|---|
| Biegefestigkeit [MPa] | 70 | 80 | 75 |
| E-Modul [MPa] | 2400 | 3500 | 2700 |
| Schlagzähigkeit [N/mm²] | 6 | 2,5 - 3 | 3 - 4 |

### Abrasionsversuche

Gebräuchlichste Methoden sind die 2-Körper-Abrasionsmessung (OCA-2-body-abrasion) und die 3-Körper-Abrasionsmessung (CFA 3-body-abrasion). Beide Methoden richten sich nach der ISO/PDTR14569/2 - Dental materials guidance on testing of wear.

### 3-Körper-Abrasion

| | **PMMA** | **Crosslinked PMMA** | **Vergleichskomposit** | **Gemäß Erfindung** |
|---|---|---|---|---|
| Depth CFA 3-body abrasion [µm] | 80 | 60 | 20 | 35 |

### 2-Körper-Abrasion (Vergleich zu Hard Resin und Composite)

| OCA 2-body-abrasion | **PMMA** | **Mitbewerber Endura (SHOFU)** | **Vergleichskomposit** | **Gemäß Erfindung** |
|---|---|---|---|---|
| Volumenverlust 120 Tsd Zyklen [mm³] | | 0,15 | 0,046 | 0,04 |
| Volumenverlust 240 Tsd Zyklen [mm³] | | 0,25 | 0,08 | 0,07 |
| Volumenverlust 480 Tsd Zyklen [mm³] | | 0,44 | 0,12 | 0,13 |
| Tiefe [mm] 120 Tsd. Zyklen | | 128 | 70 | 72 |
| Tiefe [mm] 240 Tsd. Zyklen | | 174 | 88 | 92 |
| Tiefe [mm] 480 Tsd. Zyklen | | 231 | 114 | 124 |

### 2-Körper-Abrasion (Vergleich zu kommerziellen Zähnen)

| OCA 2-body-abrasion | **PMMA** SR Orthotyp (IVOCLAR) | **PMMA vernetzt Premium** (HERAEUS KULZER) | **PMMA mit hochdispersen Füllern** NC Veracia (Shofu) | **Gemäß Erfindung** |
|---|---|---|---|---|
| Volumenverlust 20 Tsd Zyklen [mm³] | 3,5 | 0,16 | 1,25 | 0,6 |
| Volumenverlust 40 Tsd Zyklen [mm³] | 21,2 | 12,1 | 22,5 | 4 |
| Volumenverlust 100 Tsd Zyklen [mm³] | 114,6 | 84,6 | 87 | 21,2 |

Großer Volumenverlust bzw. große Tiefe bedeuten viel Abrasion. Die Werte zeigen deutlich die im Vergleich zu bekannten anorganisch gefüllten Materialien höheren Abrasionsfestigkeiten der erfindungsgemäßen Materialien. Gleichzeitig ist die Materialhärte signifikant niedriger und eher im Bereich des bekanntermaßen zähen PMMA.

### Bewertung der Vorteile

Sich aus der Verwendung der obigen Rezeptur, speziell auch der Verwendung der verstärkten Perlpolymere ergebende Vorteile sind:
■ Niedrige Härte des Materials, elastischere Struktur und hohe Zähigkeit
■ Durch gute Einbindung der in den Polymerperlen enthaltenen Füllstoffe entstehen hohe Abrasionsfestigkeiten auf dem Niveau von Compositen, herkömmlichen Zahnmaterialien weit überlegen
■ Gute Lager- und Verarbeitungseigenschaften, speziell das Mischverhalten ist dem von herkömmlichen 2-Komponenten-Systemen ähnlich. Daher resultiert ein sehr homogenes und fehlstellenfreies Material.

Durch die relativ weiche Monomermatrix und die vorab gekapselten Füllstoffe ergibt sich eine hohe Oberflächengüte des Materials.

## Patentansprüche

1. Künstlicher Zahn, der oder dessen Schneidebereich oder dessen Schmelzbereich aus einem Material mit hoher Abrasionsfestigkeit besteht, enthaltend
| | | |
|---|---|---|
| (a) | Monofunktionelle Meth-/acrylate | 25 - 30 % |
| (b) | Vernetzendes Meth-/acrylat | 6 - 10 % |
| (c) | Splitterpolymer aus Komponente (b) und (e) | 12 - 18 % |
| (d) | PMMA-Perlpolymere, teilweise vernetzt | 15 - 25 % |
| (e) | Pyrogene Kieselsäure, silanisiert | 1 - 5 % |
| (f) | Anorganisch verstärktes Perlpolymer | 20 - 30 % |
| (g) | Initiatorkomponenten | 0,1 - 1 % |
| (h) | Farbpigmente | 0,1 - 3 % |

2. Zahn nach Anspruch 1 wobei das Material enthält:
| | | |
|---|---|---|
| (a) | Monofunktionelle Meth-/acrylate | 26 - 29,8 % |
| (b) | Vernetzendes Meth-/acrylat | 8 % |
| (c) | Splitterpolymer aus Komponente (b) und (e) | 15 % |
| (d) | PMMA-Perlpolymere, teilweise vernetzt | 20 % |
| (e) | Pyrogene Kieselsäure, silanisiert | 2 % |
| (f) | Anorganisch verstärktes Perlpolymer | 25 % |
| (g) | Initiatorkomponenten | 0,1 - 1% |
| (h) | Farbpigmente | 0,1 - 3 % |

## Claims

1. Artificial tooth which or the incisal area of which or the enamel area of which consists of a material with a high abrasion resistance, containing
| | | |
|---|---|---|
| (a) | monofunctional methacrylates/acrylates | 25-30% |
| (b) | crosslinking methacrylate/acrylate | 6-10% |
| (c) | splitter polymer of the components (b) and (e) | 12-18% |
| (d) | PMMA bead polymers, partially crosslinked | 15-25% |
| (e) | pyrogenic silicic acid, silanised | 1-5% |
| (f) | inorganic reinforced bead polymer | 20-30% |
| (g) | initiator components | 0.1-1 % |
| (h) | coloured pigments | 0.1-3% |

2. Tooth according to claim 1, the material containing:
| | | |
|---|---|---|
| (a) | monofunctional methacrylates/acrylates | 26-29.8% |
| (b) | crosslinking methacrylate/acrylate | 8% |
| (c) | splitter polymer of the components (b) and (e) | 15% |
| (d) | PMMA bead polymers, partially crosslinked | 20% |
| (e) | pyrogenic silicic acid, silanised | 2% |
| (f) | inorganic reinforced bead polymer | 25% |
| (g) | initiator components | 0.1-1% |
| (h) | coloured pigments | 0.1-3% |

## Revendications

1. Dent artificielle qui ou dont le domaine tranchant ou le domaine d'émail consiste en un matériau à grande résistance à l'abrasion contenant
| | | |
|---|---|---|
| (a) | des méth/acrylates monofonctionnels | 25 - 30 % |
| (b) | un méth/acrylate réticulant | 6 - 10 % |
| (c) | un polymère en écailles des composants (b) et (e) | 12 - 18 % |
| (d) | des polymères en perles de type PMMA partiellement réticulés | 15 - 25 % |
| (e) | de l'acide silicique pyrogène silanisé | 1 - 5 % |
| (f) | un polymère en perles à renfort inorganique | 20 - 30 % |
| (g) | des composants initiateurs | 0,1 - 1 % |
| (h) | des pigments colorants | 0,1 - 3 % |

2. Dent selon la revendication 1 où le matériau contient :
| | | |
|---|---|---|
| (a) | des méth/acrylates monofonctionnels | 26 - 29,8 % |
| (b) | un méth/acrylate réticulant | 8 % |
| (c) | un polymère en écailles des composants (b) et (e) | 15 % |
| (d) | des polymères en perles de type PMMA partiellement réticulés | 20 % |
| (e) | de l'acide silicique pyrogène silanisé | 2 % |
| (f) | un polymère en perles à renfort inorganique | 25 % |
| (g) | des composants initiateurs | 0,1 - 1 % |
| (h) | des pigments colorants | 0,1 - 3 % |
